(19)

Europäisches Patentamt

European Patent Office

Office européen des brevets

(11) **EP 4 663 655 A2**

(12) **EUROPEAN PATENT APPLICATION**

(43) Date of publication:
**17.12.2025 Bulletin 2025/51**

(21) Application number: **25167189.7**

(22) Date of filing: **28.03.2025**

(51) International Patent Classification (IPC):
***C07K 16/18*** (2006.01)    ***G01N 33/68*** (2006.01)

(52) Cooperative Patent Classification (CPC):
**C07K 16/18; G01N 33/6887;** C07K 2317/33;
C07K 2317/92; G01N 2333/4712; G01N 2800/32

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.03.2024 CN 202410389129**

(71) Applicant: **Shenzhen Mindray Bio-Medical
Electronics Co., Ltd.
Shenzhen, Guangdong 518057 (CN)**

(72) Inventors:
- **AGEEVA, Ludmila V.
117186 Moscow (RU)**
- **BEREZNIKOVA, Anastasia V.
117186 Moscow (RU)**
- **BOGOMOLOVA, Agnessa P.
117186 Moscow (RU)**
- **KATRUKHA, Alexey G.
117186 Moscow (RU)**
- **KOZLOVSKY, Stanislav V.
117186 Moscow (RU)**

- **POPOVA, Anfisa S.
117186 Moscow (RU)**
- **POSTNIKOV, Alexander B.
117186 Moscow (RU)**
- **ROZOV, Fedor N.
117186 Moscow (RU)**
- **TAMM, Natalia N.
117186 Moscow (RU)**
- **ZHANG, Yi
Shenzhen, 518057 (CN)**
- **LUO, Sheng
Shenzhen, 518057 (CN)**
- **CHEN, Puguang
Shenzhen, 518057 (CN)**

(74) Representative: **KIPA AB
Drottninggatan 11
252 21 Helsingborg (SE)**

Remarks:
The complete document including Reference
Table(s) and the Sequence Listing(s) can be
downloaded from the EPO website

(54) **CARDIAC TROPONIN I SPECIFIC ANTIBODY, KIT AND USES THEREOF**

(57)    The present disclosure provides an antibody specifically targeting cardiac troponin I. The present disclosure further provides antibody pairs and kits comprising the antibodies. The present disclosure further provides uses of these antibodies to detect levels of cardiac troponin I, and to diagnose myocardial injury.

**EP 4 663 655 A2**

**Description**

CROSS REFERENCE

This application claims priority to Chinese patent application NO: CN202410389129.X, filed on March 29, 2024, the entire contents of which are incorporated herein by reference.

REFERENCE TO AN ELECTRONIC SEQUENCE LISTING

The contents of the electronic sequence listing (2401736-I-CP-SZMR.xml; Size: 73,645 bytes; and Date of Creation: Mar. 27, 2025) are herein incorporated by reference in its entirety.

TECHNICAL FIELD

The disclosure relates to the technical field of molecular immunology. In particular the disclosure relates to an antibody that specifically targets cardiac troponin I, and an antibody pair and a kit that comprises the antibody, and a use thereof.

BACKGROUND

Troponin (Tn) is a regulatory protein for muscle contraction, located on the thin filaments of contractile proteins, playing an important role in regulating muscle contraction and relaxation. Troponin has three subtypes: fast-response type, slow-response type and cardiac troponin (cTn). The first two are related to skeletal muscle, while cardiac troponin exists only in cardiac muscle cells and is composed of three subunits: troponin T(cTnT), troponin I(cTnI), and troponin C(cTnC). Cardiac troponin is degraded from cardiac muscle fibers when the cardiac muscle cells are damaged. An increase in cTn levels in blood reflects cardiomyocyte damage. Cardiac troponin I is often used to detect myocardial damage, for example, myocardial infarction, coronary sclerosis syndrome, myocarditis, pericarditis, and the like.
A method for measuring cardiac troponin I in serum is developed by Cummins et al.(Cummins et al., Am Heart Journal 113:1333-1344(1987)). However, polyclonal antibodies used in the method show significant cross-reactivity with skeletal muscle troponin I (sTnI). A sandwich ELISA method for the measurement of cardiac troponin I is developed by Bodar et al. (Bodar et al., Clinical Chemistry 38:2203-2214(1992); US Patent NO: 7,285,418), but its accuracy is low.
Therefore, there is a need for a test method that can detect cardiac troponin I with high sensitivity and accuracy, and without cross-reacting with troponin I of skeletal muscle. With such an immunological test method, treating physicians can administer appropriate treatment to provide the best possible prognosis for affected patients. The present disclosure meets these needs.

SUMMARY

In view of the foregoing, the present disclosure discloses an antibody or antigen-binding fragment thereof that specifically targets cardiac troponin I, and related compositions, reagents and methods.
In one aspect, the present disclosure provides an isolated antibody or antigen-binding fragment thereof that specifically binds to cardiac troponin I, including a heavy chain and a light chain, wherein the heavy chain includes a heavy chain variable region, and the light chain includes a light chain variable region; wherein, the heavy chain variable region comprises:

   a. CDR-H1, having an amino acid sequence selected from a group of SEQ ID NOs: 1-11NO: ;
   b. CDR-H2, having an amino acid sequence selected from a group of SEQ ID NOs: 11-22NO: ; and
   c. CDR-H3, having an amino acid sequence selected from a group of SEQ ID NOs: 23-33NO: ;

the light chain variable region comprises:

   a. CDR-L1, having an amino acid sequence selected from a group of SEQ ID NOs: 34-44NO: ;
   b. CDR-L2, having an amino acid sequence selected from a group of SEQ ID NOs: 45-55NO: ; and
   c. CDR-L3, having an amino acid sequence selected from a group of SEQ ID NOs: 56-66NO: .

In some specific embodiments, the present disclosure provides the antibody or antigen-binding fragment thereof as follows, wherein,
the heavy chain variable region comprises:

CDR-H1 as shown in SEQ ID NO: : 1, CDR-H2 as shown in SEQ ID NO: : 12, and CDR-H3 as shown in SEQ ID NO: : 23; or

CDR-H1 as shown in SEQ ID NO: 2, CDR-H2 as shown in SEQ ID NO: 13, and CDR-H3 as shown in SEQ ID NO: 24; or
CDR-H1 as shown in SEQ ID NO: 3, CDR-H2 as shown in SEQ ID NO: 14, and CDR-H3 as shown in SEQ ID NO: 25; or
CDR-H1 as shown in SEQ ID NO: 4, CDR-H2 as shown in SEQ ID NO: 15, and CDR-H3 as shown in SEQ ID NO: 26; or
CDR-H1 as shown in SEQ ID NO: 5, CDR-H2 as shown in SEQ ID NO: 16, and CDR-H3 as shown in SEQ ID NO: 27; or
CDR-H1 as shown in SEQ ID NO: 6, CDR-H2 as shown in SEQ ID NO: 17, and CDR-H3 as shown in SEQ ID NO: 28; or
CDR-H1 as shown in SEQ ID NO: 7, CDR-H2 as shown in SEQ ID NO: 18, and CDR-H3 as shown in SEQ ID NO: 29; or
CDR-H1 as shown in SEQ ID NO: 8, CDR-H2 as shown in SEQ ID NO: 19, and CDR-H3 as shown in SEQ ID NO: 30; or
CDR-H1 as shown in SEQ ID NO: 9, CDR-H2 as shown in SEQ ID NO: 20, and CDR-H3 as shown in SEQ ID NO: 31; or
CDR-H1 as shown in SEQ ID NO: 10, CDR-H2 as shown in SEQ ID NO: 21, and CDR-H3 as shown in SEQ ID NO: 32; or
CDR-H1 as shown in SEQ ID NO: 11, CDR-H2 as shown in SEQ ID NO: 22, and CDR-H3 as shown in SEQ ID NO: 33.

In some specific embodiments, the present disclosure provides the antibody or antigen-binding fragment thereof as follows, wherein,
the light chain variable region comprises:

CDR-L1 as shown in SEQ ID NO: 34, CDR-L2 as shown in SEQ ID NO: 45, and CDR-L3 as shown in SEQ ID NO: 56; or
CDR-L1 as shown in SEQ ID NO: 35, CDR-L2 as shown in SEQ ID NO: 46, and CDR-L3 as shown in SEQ ID NO: 57; or
CDR-L1 as shown in SEQ ID NO: 36, CDR-L2 as shown in SEQ ID NO: 47, and CDR-L3 as shown in SEQ ID NO: 58; or
CDR-L1 as shown in SEQ ID NO: 37, CDR-L2 as shown in SEQ ID NO: 48, and CDR-L3 as shown in SEQ ID NO: 59; or
CDR-L1 as shown in SEQ ID NO: 38, CDR-L2 as shown in SEQ ID NO: 49, and CDR-L3 as shown in SEQ ID NO: 60; or
CDR-L1 as shown in SEQ ID NO: 39, CDR-L2 as shown in SEQ ID NO: 50, and CDR-L3 as shown in SEQ ID NO: 61; or
CDR-L1 as shown in SEQ ID NO: 40, CDR-L2 as shown in SEQ ID NO: 51, and CDR-L3 as shown in SEQ ID NO: 62; or
CDR-L1 as shown in SEQ ID NO: 41, CDR-L2 as shown in SEQ ID NO: 52, and CDR-L3 as shown in SEQ ID NO: 63; or
CDR-L1 as shown in SEQ ID NO: 42, CDR-L2 as shown in SEQ ID NO: 53, and CDR-L3 as shown in SEQ ID NO: 64; or
CDR-L1 as shown in SEQ ID NO: 43, CDR-L2 as shown in SEQ ID NO: 54, and CDR-L3 as shown in SEQ ID NO: 65; or
CDR-L1 as shown in SEQ ID NO: 44, CDR-L2 as shown in SEQ ID NO: 55, and CDR-L3 as shown in SEQ ID NO: 66.

In some specific embodiments, the present disclosure provides the antibody or antigen-binding fragment thereof, wherein, the heavy chain of the antibody or antigen-binding fragment thereof comprises the three above-mentioned heavy chain CDRs, namely CDR-H1, CDR-H2, and CDR-H3.

In some specific embodiments, the present disclosure provides the antibody or antigen-binding fragment thereof, wherein, the light chain of the antibody or antigen-binding fragment thereof comprises three above-mentioned light chain CDRs, namely CDR-L1, CDR-L2, and CDR-L3.

In some specific embodiments, the present disclosure provides the antibody or antigen-binding fragment thereof, which comprises:

CDR-H1 as shown in SEQ ID NO: 1, CDR-H2 as shown in SEQ ID NO: 12, and CDR-H3 as shown in SEQ ID NO: 23; and CDR-L1 as shown in SEQ ID NO: 34, CDR-L2 as shown in SEQ ID NO: 45, and CDR-L3 as shown in SEQ ID NO: 56; or
CDR-H1 as shown in SEQ ID NO: 2, CDR-H2 as shown in SEQ ID NO: 13, and CDR-H3 as shown in SEQ ID NO: 24; and CDR-L1 as shown in SEQ ID NO: 35, CDR-L2 as shown in SEQ ID NO: 46, and CDR-L3 as shown in SEQ ID NO: 57; or
CDR-H1 as shown in SEQ ID NO: 3, CDR-H2 as shown in SEQ ID NO: 14, and CDR-H3 as shown in SEQ ID NO: 25; and CDR-L1 as shown in SEQ ID NO: 36, CDR-L2 as shown in SEQ ID NO: 47, and CDR-L3 as shown in SEQ ID NO: 58; or
CDR-H1 as shown in SEQ ID NO: 4, CDR-H2 as shown in SEQ ID NO: 15, and CDR-H3 as shown in SEQ ID NO: 26; and CDR-L1 as shown in SEQ ID NO: 37, CDR-L2 as shown in SEQ ID NO: 48, and CDR-L3 as shown in SEQ ID NO: 59; or
CDR-H1 as shown in SEQ ID NO: 5, CDR-H2 as shown in SEQ ID NO: 16, and CDR-H3 as shown in SEQ ID NO: 27; and CDR-L1 as shown in SEQ ID NO: 38, CDR-L2 as shown in SEQ ID NO: 49, and CDR-L3 as shown in SEQ ID NO: 60; or
CDR-H1 as shown in SEQ ID NO: 6, CDR-H2 as shown in SEQ ID NO: 17, and CDR-H3 as shown in SEQ ID NO: 28; and CDR-L1 as shown in SEQ ID NO: 39, CDR-L2 as shown in SEQ ID NO: 50, and CDR-L3 as shown in SEQ ID NO: 61; or
CDR-H1 as shown in SEQ ID NO: 7, CDR-H2 as shown in SEQ ID NO: 18, and CDR-H3 as shown in SEQ ID NO: 29;

and CDR-L1 as shown in SEQ ID NO: 40, CDR-L2 as shown in SEQ ID NO: 51, and CDR-L3 as shown in SEQ ID NO: 62; or

CDR-H1 as shown in SEQ ID NO: 8, CDR-H2 as shown in SEQ ID NO: 19, and CDR-H3 as shown in SEQ ID NO: 30; and CDR-L1 as shown in SEQ ID NO: 41, CDR-L2 as shown in SEQ ID NO: 52, and CDR-L3 as shown in SEQ ID NO: 63; or

CDR-H1 as shown in SEQ ID NO: 9, CDR-H2 as shown in SEQ ID NO: 20, and CDR-H3 as shown in SEQ ID NO: 31; and CDR-L1 as shown in SEQ ID NO: 42, CDR-L2 as shown in SEQ ID NO: 53, and CDR-L3 as shown in SEQ ID NO: 64; or

CDR-H1 as shown in SEQ ID NO: 10, CDR-H2 as shown in SEQ ID NO: 21, and CDR-H3 as shown in SEQ ID NO: 32; and CDR-L1 as shown in SEQ ID NO: 43, CDR-L2 as shown in SEQ ID NO: 54, and CDR-L3 as shown in SEQ ID NO: 65; or

CDR-H1 as shown in SEQ ID NO: 11, CDR-H2 as shown in SEQ ID NO: 22, and CDR-H3 as shown in SEQ ID NO: 33; and CDR-L1 as shown in SEQ ID NO: 44, CDR-L2 as shown in SEQ ID NO: 55, and CDR-L3 as shown in SEQ ID NO: 66.

In some specific embodiments, the antibody or antigen-binding fragment thereof has a KD value of less than 20 nM. For example, KD value is less than 15nM, 12nM, 10nM, 8nM, 5nM, 4nM, 3nM, 2nM, 1nM, 0.5nM. Preferably, the KD value is measured by biofilm interference technology.

In some specific embodiments, the antibody is a monoclonal antibody.

In some specific embodiments, the antibody or antigen-binding fragment thereof comprises non-CDR regions.

In some specific embodiments, the non-CDR regions may or may not be homologous to the CDR regions.

In some specific embodiments, the antibody or antigen-binding fragment thereof is selected from the group comprising Fab, Fab', F(ab')$_2$, Fd, Fv, dAb, complementarity determining regions fragments, single chain antibodies (e.g., scFvs), humanized antibodies, chimeric antibodies, or diabodies.

In another aspect, the present disclosure provides an isolated nucleic acid molecule comprising a nucleotide sequence encoding the antibody or antigen-binding fragment thereof as described above.

In some specific embodiments, the nucleic acid molecule comprises nucleic acid molecule A, which: encodes the heavy chain of the above-mentioned antibody or antigen-binding fragment thereof, wherein the heavy chain includes CDR-H1, CDR-H2, and CDR-H3.

In some specific embodiments, the nucleic acid molecule comprises nucleic acid molecule B, which: encodes the light chain of the above-mentioned antibody or antigen-binding fragment thereof, wherein the light chain includes CDR-L1, CDR-L2, and CDR-L3.

In some specific embodiments, the nucleic acid molecule comprises nucleic acid molecule C, which: the above-mentioned nucleic acid molecule A, and the above-mentioned nucleic acid molecule B; optionally, the nucleic acid molecule C further includes a linker sequence, which is used to connect the nucleic acid molecule A and the nucleic acid molecule B.

In another aspect, the present disclosure provides a vector comprising the aforementioned nucleic acid molecule.

In some specific embodiments, the vector includes the nucleic acid molecule A, the nucleic acid molecule B, or the nucleic acid molecule C as described above.

In another aspect, the present disclosure provides a host cell comprising the above-mentioned nucleic acid molecule or the vector.

In some specific embodiments, the host cell comprises the above-mentioned nucleic acid molecule A, nucleic acid molecule B, nucleic acid molecule C, or the vector.

In another aspect, the present disclosure provides a conjugate comprising a monoclonal antibody or antigen-binding fragment thereof and a coupling moiety, wherein the monoclonal antibody is the antibody or antigen-binding fragment thereof of the present disclosure, the coupling moiety is a detectable label; preferably, the coupling moiety is a radioisotope, a fluorescent substance, a luminescent substance, a colored substance or an enzyme.

In another aspect, the present disclosure provides an antibody pair comprising a capture antibody and a detection antibody, wherein the capture antibody and the detection antibody target different epitopes of cardiac troponin I, wherein the capture antibody and the detection antibody are selected from the group consisting of the antibody or antigen-binding fragment thereof as described above, Known Antibody 1 (KA1), Known Antibody 2 (KA2), or Known Antibody 3 (KA3).

In some specific embodiments, KA1 is an antibody which comprises CDR-H1~3 as shown in SEQ ID NOs: 67-69; and CDR-L1~3 as shown in SEQ ID NOs: 70~72.

In some specific embodiments, KA2 is an antibody which comprises CDR-H1~3 as shown in SEQ ID NOs: 73-75; and CDR-L1~3 as shown in SEQ ID NOs: 76~78.

In some specific embodiments, KA3 is an antibody which comprises CDR-H1~3 as shown in SEQ ID NOs: 79-81;

and CDR-L1~3 as shown in SEQ ID NOs: 82~84.

The capture antibody and the detection antibody can be interchangeable, that is, one antibody can be used as a detection antibody, and also can be used as a capture antibody.

Preferably, the antibody pair includes a capture antibody and a detection antibody, and the capture antibody is an antibody which comprises CDR-H1~3 as shown in SEQ **ID** NOs: 73-75; and CDR-L1~3 as shown in SEQ **ID** NOs: 76-78; the detection antibody is an antibody which comprises CDR-H1 having an amino acid sequence of SEQ ID NO: 9 and CDR-H2 having an amino acid sequence of SEQ ID NO: 20, and CDR-H3 having an amino acid sequence of SEQ ID NO: 31; and CDR-L1 having an amino acid sequence of SEQ ID NO: 42, CDR-L2 having an amino acid sequence of SEQ ID NO: 53, and CDR-L3 having an amino acid sequence of SEQ ID NO: 64.

Preferably, the antibody pair includes a capture antibody and a detection antibody, and the capture antibody is an antibody which comprises CDR-H1~3 as shown in SEQ **ID** NOs: 73-75; and CDR-L1~3 as shown in SEQ **ID** NOs: 76-78; the detection antibody is an antibody which comprises CDR-H1 having an amino acid sequence of SEQ ID NO: 6 and CDR-H2 having an amino acid sequence of SEQ ID NO: 17, and CDR-H3 having an amino acid sequence of SEQ ID NO: 28; and CDR-L1 having an amino acid sequence of SEQ ID NO: 39, CDR-L2 having an amino acid sequence of SEQ ID NO: 50, and CDR-L3 having an amino acid sequence of SEQ ID NO: 61.

Preferably, the antibody pair includes a capture antibody and a detection antibody, and the capture antibody is an antibody which comprises CDR-H1~3 as shown in SEQ **ID** NOs: 67-69; and CDR-L1~3 as shown in SEQ **ID** NOs: 70-72; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60.

Preferably, the antibody pair includes a capture antibody and a detection antibody, and the capture antibody is an antibody which comprises CDR-H1~3 as shown in SEQ **ID** NOs: 79-81; and CDR-L1~3 as shown in SEQ **ID** NOs: 82-84; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

Preferably, the antibody pair includes a capture antibody and a detection antibody, and the capture antibody is an antibody which comprises CDR-H1~3 as shown in SEQ ID NOs: 79-81; and CDR-L1~3 as shown in SEQ ID NOs: 82-84; the detection antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66.

Preferably, the antibody pair includes a capture antibody and a detection antibody, and the capture antibody is an antibody which comprises CDR-H1 having an amino acid sequence of SEQ ID NO: 3 and CDR-H2 having an amino acid sequence of SEQ ID NO: 14, and CDR-H3 having an amino acid sequence of SEQ ID NO: 25; and CDR-L1 having an amino acid sequence of SEQ ID NO: 36, CDR-L2 having an amino acid sequence of SEQ ID NO: 47, and CDR-L3 having an amino acid sequence of SEQ ID NO: 58; the detection antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66.

Preferably, the antibody pair includes a capture antibody and a detection antibody, and the capture antibody is an antibody which comprises CDR-H1 having an amino acid sequence of SEQ ID NO: 4 and CDR-H2 having an amino acid sequence of SEQ ID NO: 15, and CDR-H3 having an amino acid sequence of SEQ ID NO: 26; and CDR-L1 having an amino acid sequence of SEQ ID NO: 37, CDR-L2 having an amino acid sequence of SEQ ID NO: 48, and CDR-L3 having an amino acid sequence of SEQ ID NO: 59; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60.

Preferably, the antibody pair includes a capture antibody and a detection antibody, and the capture antibody is an antibody which comprises CDR-H1 having an amino acid sequence of SEQ ID NO: 1 and CDR-H2 having an amino acid sequence of SEQ ID NO: 12, and CDR-H3 having an amino acid sequence of SEQ ID NO: 23; and CDR-L1 having an amino acid sequence of SEQ ID NO: 34, CDR-L2 having an amino acid sequence of SEQ ID NO: 45, and CDR-L3 having an amino acid sequence of SEQ ID NO: 56; the detection antibody is an antibody which comprises CDR-H1 having an amino acid sequence of SEQ ID NO: 9 and CDR-H2 having an amino acid sequence of SEQ ID NO: 20, and CDR-H3 having an amino acid sequence of SEQ ID NO: 31; and CDR-L1 having an amino acid sequence of SEQ ID NO: 42, CDR-L2 having an amino acid sequence of SEQ ID NO: 53, and CDR-L3 having an amino acid sequence of SEQ ID NO: 64.

Preferably, the antibody pair includes a capture antibody and a detection antibody, and the capture antibody is an antibody which comprises CDR-H1 having an amino acid sequence of SEQ ID NO: 3 and CDR-H2 having an amino acid sequence of SEQ ID NO: 14, and CDR-H3 having an amino acid sequence of SEQ ID NO: 25; and CDR-L1 having an amino acid sequence of SEQ ID NO: 36, CDR-L2 having an amino acid sequence of SEQ ID NO: 47, and CDR-L3 having an amino acid sequence of SEQ ID NO: 58; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60.

Preferably, the antibody pair includes a capture antibody and a detection antibody, and the capture antibody is an antibody which comprises CDR-H1 having an amino acid sequence of SEQ ID NO: 2 and CDR-H2 having an amino acid sequence of SEQ ID NO: 13, and CDR-H3 having an amino acid sequence of SEQ ID NO: 24; and CDR-L1 having an amino acid sequence of SEQ ID NO: 35, CDR-L2 having an amino acid sequence of SEQ ID NO: 46, and CDR-L3 having an amino acid sequence of SEQ ID NO: 57; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

Preferably, the antibody pair includes a capture antibody and a detection antibody, and the capture antibody is an antibody which comprises CDR-H1 having an amino acid sequence of SEQ ID NO: 10 and CDR-H2 having an amino acid sequence of SEQ ID NO: 21, and CDR-H3 having an amino acid sequence of SEQ ID NO: 32; and CDR-L1 having an amino acid sequence of SEQ ID NO: 43, CDR-L2 having an amino acid sequence of SEQ ID NO: 54, and CDR-L3 having an amino acid sequence of SEQ ID NO: 65; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

Preferably, the antibody pair includes a capture antibody and a detection antibody, and the capture antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

Preferably, the antibody pair includes a capture antibody and a detection antibody, and the capture antibody is an antibody which comprises CDR-H1 having an amino acid sequence of SEQ ID NO: 1 and CDR-H2 having an amino acid sequence of SEQ ID NO: 12, and CDR-H3 having an amino acid sequence of SEQ ID NO: 23; and CDR-L1 having an amino acid sequence of SEQ ID NO: 34, CDR-L2 having an amino acid sequence of SEQ ID NO: 45, and CDR-L3 having an amino acid sequence of SEQ ID NO: 56; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

In yet another aspect, the present disclosure provides a kit, comprising the antibody or antigen-binding fragment thereof, or the conjugate thereof.

Preferably, the kit further comprises a second antibody, which specifically recognizes the antibody or its antigen-binding fragment; optionally, the second antibody further includes a detectable label, such as a radioactive isotope, a fluorescent substance, a luminescent substance, a coloured substance or an enzyme.

The present disclosure provides a kit comprising the antibody pair of the present disclosure; optionally, the detection antibody includes a detectable label, such as a radioisotope, a fluorescent substance, a luminescent substance, a colored substance or an enzyme.

In another aspect, the present disclosure provides the use of the antibody or antigen-binding fragment thereof in any embodiment of the present disclosure or the antibody pair of the present disclosure or the conjugate of the present disclosure in the preparation of a kit for detecting the presence or level of cardiac troponin I in a sample from a subject.

In a specific embodiment, the present disclosure provides the use of the antibody or antigen-binding fragment thereof of any embodiment of the present disclosure or the antibody pair of the present disclosure or the conjugate of the present disclosure in the preparation of a kit for assisting diagnosis of myocardial injury.

In some specific embodiments, the myocardial injury is caused by myocardial infarction, coronary sclerosis syndrome, myocarditis, pericarditis, and the like.

In a specific embodiment, the present disclosure provides the use of the antibody or antigen-binding fragment thereof of any embodiment of the present disclosure or the antibody pair of the present disclosure or the conjugate of the present disclosure in the preparation of a kit for assisting diagnosis of myocardial infarction.

In another aspect, the present disclosure provides a method for producing the antibody or antigen-binding fragment thereof of the present disclosure, comprising culturing the host cell of the present disclosure under a suitable condition, and recovering the antibody or antigen-binding fragment thereof from the cell culture.

In another aspect, the present disclosure provides the antibody or antigen-binding fragment thereof, comprising the antibody or antigen-binding fragment thereof which are obtained by the above method for producing the antibody or antigen-binding fragment thereof.

In some specific embodiments, the present disclosure provides the antibody or antigen-binding fragment thereof, comprising culturing the *Escherichia coli* of the present disclosure, obtaining the molecular genetic construct, and transferring the molecular genetic construct to the host cell, and culturing the host cell to recover the antibody or antigen-binding fragment thereof from the cell culture.

The molecular genetic construct may be a single molecular genetic construct encoding the full sequence of the antibody, such as the molecular genetic construct obtained by culturing *Escherichia coli* with the deposit number of VKPM B-14631, etc.

The molecular genetic construct may also be two molecular genetic constructs encoding the antibody light chain sequence and the antibody heavy chain sequence, respectively. For example, molecular genetic constructs obtained by culturing *Escherichia coli* with deposit number of VKPM B-14668 and VKPM B-14669.

In yet another aspect, the present disclosure provides a method for assisting diagnosis of myocardial injury, comprising the step of administering the antibody or antigen-binding fragment thereof of the present disclosure to a sample from a subject.

An antibody or antigen-binding fragment thereof that specifically binds to cardiac troponin I, wherein the antibody or antigen-binding fragment thereof is:

produced based on *Escherichia coli* Rosetta™(DE3)pLysS the plasmid of new antibody2 (NA2) deposited with the All-Russian National Collection of Industrial Microorganisms (VKPM) having Accession Number B-14631 on December 14, 2023;

produced based on *Escherichia coli* Rosetta™(DE3)pLysS 3 (NA3) VL deposited with VKPM having Accession Number B-14669 on February 7, 2024, and *Escherichia coli* RosettaTM(DE3)pLysS 3 (NA3) VH deposited with VKPM having Accession Number B-14668 on February 7, 2024;

produced based on *Escherichia coli* Rosetta™(DE3)pLysS 4 (NA4) VL deposited with VKPM having Accession Number B-14671 on February 7, 2024, and *Escherichia coli* RosettaTM(DE3)pLysS 4 (NA4) VH deposited with VKPM having Accession Number B-14670 on February 7, 2024;

produced based on *Escherichia coli* Rosetta™(DE3)pLysS 5 (NA5) VL deposited with VKPM having Accession Number B-14673 on February 7, 2024, and *Escherichia coli* RosettaTM(DE3)pLysS 5 (NA5) VH deposited with VKPM having Accession Number B-14672 on February 7, 2024;

produced based on *Escherichia coli* Rosetta™(DE3)pLysS 6 (NA6) VL deposited with VKPM having Accession Number B-14675 on February 7, 2024, and *Escherichia coli* RosettaTM(DE3)pLysS 6 (NA6) VH deposited with VKPM having Accession Number B-14674 on February 7, 2024;

produced based on *Escherichia coli* Rosetta™(DE3)pLysS 7 (NA7) VL deposited with VKPM having Accession Number B-14677 on February 7, 2024, and *Escherichia coli* RosettaTM(DE3)pLysS 7 (NA7) VH deposited with VKPM having Accession Number B-14676 on February 7, 2024;

produced based on *Escherichia coli* Rosetta™(DE3)pLysS 8 (NA8) VL deposited with VKPM having Accession Number B-14679 on February 7, 2024, and *Escherichia coli* RosettaTM(DE3)pLysS 8 (NA8) VH deposited with VKPM having Accession Number B-14678 February 7, 2024;

produced based on *Escherichia coli* Rosetta™(DE3)pLysS the plasmid of new antibody 9 (NA9) deposited with the VKPM having Accession Number B-14632 on December 14, 2023;

produced based on *Escherichia coli* Rosetta™(DE3)pLysS the plasmid of new antibody 10 (NA10) deposited with the VKPM having Accession Number B-14633 on December 14, 2023;

produced based on *Escherichia coli* Rosetta™(DE3)pLysS 11 (NA11) VL deposited with VKPM having Accession Number B-14681 on February 7, 2024, and *Escherichia coli* RosettaTM(DE3)pLysS 11 (NA11) VH deposited with VKPM having Accession Number B-14680 on February 7, 2024;

produced based on *Escherichia coli* Rosetta™(DE3)pLysS 12 (NA12) VL deposited with VKPM having Accession Number B-14683 on February 7, 2024, and *Escherichia coli* RosettaTM(DE3)pLysS 12 (NA12) VH deposited with VKPM having Accession Number B-14682 on February 7, 2024.

BRIEF DESCRIPTION OF THE DRAWINGS

FIG. 1 shows the results of the measurement performance of the antibody pair of the present disclosure to different types of samples.

FIG. 2 shows the results of the prognostic performance of the antibody pair of the present disclosure of patient 1 with acute myocardial injury.

FIG. 3 shows the results of the prognostic performance of the antibody pair of the present disclosure of patient 2 with acute myocardial injury.

FIG. 4 shows the results of the prognostic performance of the antibody pair of the present disclosure of patient 3 with acute myocardial injury.

DETAILED DESCRIPTION

In the present disclosure, unless otherwise indicated, the scientific and technical terms used in this disclosure shall have the meaning commonly understood by a person skilled in the art. In addition, steps of the cell culture, molecular genetics, nucleic acid chemistry, and immunology related laboratory procedures used in this disclosure are the general procedures used in the relevant fields. Meanwhile, in order to better understand the disclosure, the definitions and interpretations of relevant terms are provided below.

Unless otherwise indicated, the term "isolated molecule" (where the molecule is, for example, a polypeptide, a polynucleotide, or an antibody or fragment thereof) is a molecule that by virtue of its origin or source of derivation, (1) is not associated with naturally associated components that accompany it in its native state, (2) is substantially free of other molecules from the same species (3) is expressed by a cell from a different species, or (4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

An "antibody" is an immunoglobulin molecule capable of specific binding to a target, such as a carbohydrate, polynucleotide, lipid, polypeptide, or the like, through at least one antigen recognition site, located in the variable region of the immunoglobulin molecule. As used herein, the term encompasses not only intact polyclonal or monoclonal antibodies, but also, unless otherwise specified, any antigen binding portion thereof that competes with the intact antibody for specific binding, fusion proteins comprising an antigen binding portion, and any other modified configuration of the immunoglobulin molecule that comprises an antigen recognition site. Antigen binding portions include, for example, Fab, Fab', $F(ab')_2$, Fd, Fv, domain antibodies (dAbs, e.g., shark and camelid antibodies), fragments including complementarity determining regions (CDRs), single chain variable fragment antibodies (scFv), maxibodies, minibodies, intrabodies, diabodies, triabodies, tetrabodies, v-NAR and bis-scFv, and polypeptides that contain at least a portion of an immunoglobulin that is sufficient to confer specific antigen binding to the polypeptide. An antibody includes an antibody or antigen-binding fragment thereof of any class, such as IgG, IgA, or IgM (or sub-class thereof), and the antibody need not be of any particular class. Depending on the antibody amino acid sequence of heavy chain constant region of immunoglobulins, the immunoglobulins can be different classes of immunoglobulins. There are five major classes of immunoglobulins: IgA, IgD, IgE, IgG, and IgM, and several of these immunoglobulins may be further divided into subclasses (isotypes), e.g., IgG1, IgG2, IgG3, IgG4, IgA1 and IgA2. The heavy-chain constant regions that correspond to the different classes of immunoglobulins are called alpha $(\alpha)$, delta $(\delta)$, epsilon $(\varepsilon)$, gamma $(\gamma)$, and mu $(\mu)$, respectively. The subclass structures and three-dimensional configurations of different classes of immunoglobulins are well known.

The terms "antigen-binding portion" or "antigen-binding fragment" of an antibody (or simply "antibody portion"), as used interchangeably herein, refers to one or more fragments of an antibody that retain the ability to specifically bind to an antigen (e.g., cardiac troponin). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a $F(ab')_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR), disulfide-linked Fvs (dsFv), and anti-idiotypic (anti-Id) antibodies and intrabodies. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv)). Such single chain antibodies are also intended to be encompassed within the term "antigen-binding portion" of an antibody. Other forms of single chain antibodies, such as diabodies, are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites.

Antibodies may be derived from any mammal, including, but not limited to, humans, monkeys, pigs, horses, rabbits, dogs, cats, mice, or other animals such as birds (e.g., chickens), fish (e.g., sharks) and camelids (e.g., llamas).

A "variable region" of an antibody refers to the variable region of the antibody light chain (VL) or the variable region of the antibody heavy chain (VH), either alone or in combination. As known in the art, the variable regions of the heavy and light chains each consist of four framework regions (FRs) connected by three complementarity determining regions (CDRs) also known as hypervariable regions, and contribute to the formation of the antigen binding site of antibodies. If variants of a subject variable region are desired, particularly with substitution in amino acid residues outside of a CDR region (i.e., in the framework region), appropriate amino acid substitution, preferably, conservative amino acid substitution, can be identified by comparing the subject variable region to the variable regions of other antibodies that contain CDR1 and CDR2 sequences in the same canonical class as the subject variable region.

A variant antibody may comprise 1, 2, 3, 4, 5, up to 10, up to 20, up to 30 or more amino acid substitutions and/or deletions and/or insertions from the specific sequences and fragments discussed above. "Deletion" variants may comprise the deletion of individual amino acids, deletion of small groups of amino acids such as 2, 3, 4 or 5 amino acids, or deletion of larger amino acid regions, such as the deletion of specific amino acid domains or other features. "Insertion" variants may comprise the insertion of individual amino acids, insertion of small groups of amino acids such as 2, 3, 4 or 5 amino acids, or insertion of larger amino acid regions, such as the insertion of specific amino acid domains or other features. "Substitution" variants preferably involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid or another aliphatic amino acid.

As mentioned in the context, certain positions of the antibody molecule can be altered. "Position" as used herein is meant a location in the sequence of a protein. Positions may be numbered sequentially, or according to an established format, for example the EU index and Kabat index can be used to number amino acid residues of an antibody. The positions as discussed above are generally determined by alignment with other parent sequences.

Generally, the term "epitope" refers to an area or a region of an antigen to which an antibody specifically binds, e.g., an area or a region comprising a contact residue that interacts with the antibody. Thus, the term "epitope" refers to that portion of a molecule capable of being recognized by and bound by an antibody at one or more of the antibody's antigen-binding regions. Typically, an epitope is defined in the context of a molecular interaction between an antibody, or antigen-binding fragment thereof, and its corresponding antigen. Epitopes often consist of a surface grouping of molecules such as amino acids or sugar side chains and have specific three-dimensional structural characteristics as well as specific charge characteristics.

The terms "polypeptide", "oligopeptide", "peptide" and "protein" are used interchangeably herein to refer to chains of amino acids of any length. The chain may be linear or branched, it may comprise modified amino acids, and/or may be interrupted by non-amino acids. The terms also encompass an amino acid chain that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that the polypeptides can occur as single chains or associated chains.

The term "dissociation constant" is sometimes used interchangeably with "equilibrium dissociation constant", and refers to the value obtained in a titration measurement at equilibrium, or by dividing the dissociation rate constant (Koff) by the association rate constant (Kon). The association rate constant, the dissociation rate constant and the equilibrium dissociation constant are used to represent the binding affinity of an antibody to an antigen. Methods for determining association and dissociation rate constants are well known in the art.

Binding affinity between two molecules, e.g. an antibody or fragment thereof, and an antigen, through a monovalent interaction may be quantified by determination of the dissociation constant (KD). In turn, KD can be determined by measurement of the kinetics of complex formation and dissociation using, e.g., the surface plasmon resonance (SPR) method (Biacore). The rate constants corresponding to the association and the dissociation of a monovalent complex are referred to as the association rate constants Ka (or Kon) and dissociation rate constant Kd (or Koff), respectively. KD is related to Ka and Kd through the equation KD=Kd/Ka. The value of the dissociation constant can be determined directly by well-known methods, and can be computed even for complex mixtures by methods such as those, for example, set forth in Caceci et al. (1984, Byte 9: 340-362).

As used herein, "vector" means a construct, which is capable of delivering, and, preferably, expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

An "individual" or a "subject" refers to a mammal, more preferably, a human. Mammals also include, but are not limited to, farm animals (e.g., cows, pigs, horses, chickens, etc.), sport animals, pets, primates, horses, dogs, cats, mice and rats.

Hereinafter, the present disclosure will be described in detail with reference to specific embodiments and examples, and the advantages and various effects of the present disclosure will be more clearly presented therefrom. Those skilled in the art should understand that these specific embodiments and examples are used to illustrate the present disclosure, rather than limit the present disclosure.

Example 1. Screening of anti-cardiac troponin I antibodies

Recombinant soluble cardiac troponin I was expressed in E. coli and further purified. The resulting purified protein solution was concentrated, stored below -80°C, and the purity was confirmed by SDS-PAGE and molecular sieve chromatography (SEC).

Recombinant soluble cardiac troponin I was used to immunize rabbits, rats, and pigs. The immunized hybridoma cells were screened to obtain 11 hybridoma cells that produced anti-cardiac troponin I (the antibodies produced were named new antibody (NA)): NA2, NA3, NA4, NA5, NA6, NA7, NA8, NA9, NA10, NA11, and NA12, where NA2, NA3, NA4, and NA5 are of rabbit origin, NA6, NA7, NA8, NA9, and NA10 are of rat origin, and NAT11 and NA12 are of pig origin). RNA was extracted from the screened hybridoma cells and their antibody-producing sequences were sequenced. Partial sequencing results are shown in Table 1 (amino acid sequence) below.

Table 1

| Antibody name | Heavy chain | | |
|---|---|---|---|
| | CDR1 (SEQ ID NO: 1-11) | CDR2 (SEQ ID NO: 12-22) | CDR3 (SEQ ID NO: 23-33) |
| NA2 | SSYWMC | CIYGGRSGSTYYANWLNG | ARRDDDGTHSLRL |
| NA3 | GNPMC | CISSFSNNNDYANWAKG | ARGLGDTNL |
| NA4 | SSVMC | CVHGGDGSTDYATWAKG | TRDFDL |
| NA5 | YKYMC | CIFAGLFDNIYYTTWARG | ARDPAGATDL |
| NA6 | NYYMV | SISDDGGSTYYRDSVKG | ATGGFDY |
| NA7 | NYDMA | SISHGGNDTAYRDSVEG | TRGGYWFPY |
| NA8 | DYWMS | DIKYDGSSTNYAPSLLN | TRIGGTYYFDY |
| NA9 | NNYMS | IINLGTGETSYNVKFTG | ASLGIRGRNYVMYA |
| NA10 | DTGVN | AIWSGGDTDYNSTLKS | ALLRGSRFDS |
| NA11 | DYSVT | TRYPSGTTYDADSVKG | IVGFFL |
| NA12 | SKYIN | GITTRSSNTAYSDSVKG | ATSALTL |
| | Light chain | | |
| | CDR1 (SEQ ID NO: 34-44) | CDR2 (SEQ ID NO: 45-55) | CDR3 (SEQ ID NO: 56-66) |
| NA2 | QASERISIYLA | DTSKLAS | CQSTYGIRDGN |
| NA3 | QSSQSVHGSDWLS | TTSTLAS | LGGYDTDIDTYG |
| NA4 | QSSQSVYNNNWLN | RASTLAS | LAAYVDDGDTYG |
| NA5 | QASQSVYDNRYLS | DASTLAS | AGYKSYNDDDYG |
| NA6 | RSSHSLLDSTGNTYLY | SVSNLGS | MEAPHGPFT |
| NA7 | RSSQSLIHRNGNIYLN | RVSNRFS | LQGTHLPYT |
| NA8 | RSSQSLEHPDGNTHLS | RVSNRFS | LQSTRFPYT |
| NA9 | RSSQSLVYSDGDTFLD | EVSNRFS | FQATHFPFT |
| NA10 | RSSKNFVHSDGNTYLN | KVSNRLS | GQVSKIPLT |

(continued)

| | Light chain | | |
|---|---|---|---|
| | CDR1 (SEQ ID NO: 34-44) | CDR2 (SEQ ID NO: 45-55) | CDR3 (SEQ ID NO: 56-66) |
| NA11 | RSSQSLLYTNGKKYLN | YATNRDI | FQNLQSPYG |
| NA12 | RSSQSLLDTDGENYLN | DATKWAS | WQSFKSPFD |

Example 2. Production of anti-cardiac troponin I antibodies

Antibodies, new antibody 2(NA2), new antibody 3(NA3), new antibody 4(NA4), new antibody 5(NA5), new antibody 6(NA6), new antibody 7(NA7), new antibody 8(NA8), new antibody 9(NA9), new antibody 10(NA10), new antibody 11(NA11), new antibody 12(NA12) were obtained in recombinant form. The design of appropriate molecular genetic constructs was carried. To develop molecular genetic constructs of rat and porcine antibodies, variable domains of the NA6, NA7, NA8, NA9, NA10, NA11, NA12 antibodies and constant domains of human immunoglobulins of the IgG1 isotype for the heavy chain and the lambda isotype for the light chain were used. To develop molecular genetic constructs of rabbit antibodies, variable domains of the NA2, NA3, NA4, NA5 antibodies and constant domains of rabbit immunoglobulins of the IgG isotype for the heavy chain and the lambda isotype for the light chain were used.

Molecular genetic constructs of recombinant antibodies were obtained in preparative quantities in bacterial cells and purified.

Mammalian cell lines Expi293F were transfected using molecular genetic constructs of recombinant antibodies. For example, by means of homologous recombination or restriction enzyme digestion, light chain and heavy chain gene sequences were transferred to the Open Reading Frame (ORF) of the expression vector seperately, and finally the structures of "promoter-antibody light chain gene-terminator" and "promoter-antibody heavy chain gene-terminator" were formed on the two expression vectors respectively. Then, by means of biological, physical and chemical methods, the two expression vectors (antibody light chain expression vector and heavy chain expression vector) were simultaneously transferred into the mammalian cell line Expi293F for expression. Finally, two light chains and two heavy chains were reassembled into an intact antibody in the cell and secreted into the culture supernatant. The expression vector may also be transformed to contain two Open Reading Frames, and then the light chain and heavy chain gene sequences were inserted into the two Open Reading Frames (ORF) of the expression vector through homologous recombination or restriction enzyme digestion, forming a structure of "promoter-antibody light chain gene-terminator-vector sequence-promoter-antibody heavy chain gene-terminator" or "promoter-antibody heavy chain gene-terminator-vector sequence-promoter-antibody light chain gene-terminator". The expression vector containing both light chain and heavy chain of the antibody was transferred into the mammalian cell line Expi293F for expression by biological, physical and chemical methods. Finally, two light chains and two heavy chains were reassembled into an intact antibody in the cell and secreted into the culture supernatant.

Antibodies were purified from conditioned culture medium (NA2, NA3, NA4, NA5, NA6, NA7, NA8, NA9, NA10, NA11, and NA12) by using Protein A affinity chromatography. The resin was from GE Healthcare Life Sciences (Piscataway, NJ), and purification was carried out according to manufacturer's instructions. Purified monoclonal antibodies were stored as suspensions in 50% ammonium sulfate at 4°C.

Example 3. Measurement of the affinity of antibodies

Each of the antibodies prepared in Example 2 was dissolved in PBS(5μg/mL), and was applied to the sensor, and the sensor was blocked. Antigen (cardiac troponin complex) was then administered at concentrations of 10, 30 and 90 nM, respectively. Finally, the affinity of the antibody was measured according to biofilm interference, and the measurement results are shown in Table 2. It can be seen from Table 2 that the 11-strain antibodies of the present disclosure all have good affinity for the target cardiac troponin.

Table 2

| Antibody | Ka(1/Ms) | Kd(1/s) | KD(M) |
|---|---|---|---|
| NA2 | 1.11E+05 | 1.33E-03 | 1.38E-08 |
| NA3 | 1.63E+05 | 5.64E-04 | 3.70E-09 |
| NA4 | 1.77E+05 | 2.16E-04 | 1.23E-09 |
| NA5 | 1.10E+05 | 3.26E-04 | 2.95E-09 |

(continued)

| Antibody | Ka(1/Ms) | Kd(1/s) | KD(M) |
|----------|----------|---------|-------|
| NA6 | 1.18E+05 | 4.50E-04 | 3.85E-09 |
| NA7 | 8.82E+04 | 4.75E-04 | 5.78E-09 |
| NA8 | 1.24E+05 | 1.50E-03 | 1.20E-08 |
| NA9 | 1.35E+05 | 2.37E-04 | 1.97E-09 |
| NA10 | 9.11E+04 | 4.10E-04 | 4.76E-09 |
| NA11 | 1.56E+05 | 1.71E-03 | 1.09E-08 |
| NA12 | 6.99E+04 | 7.23E-04 | 1.08E-08 |

Example 4. Testing of Epitope specificity of antibodies

Epitope specificity of monoclonal antibodies was determined by ELISA with short peptides (15-20 amino acids, derived from protein sequence 18-200 amino acids). Peptides were conjugated with BSA by using sulfo-SMCC obtained from Pierce (Rockford, IL) according to manufacturer's instructions. BSA was linked to peptides by one additional cysteine residue from the N-terminus. For the conjugation 2.5 mg of carrier protein - bovine serum albumin (BSA) (obtained from Sigma Chemicals, St. Louise, Mo.) was dissolved in PBS to the concentration 10 mg/ml. Two milligrams of sulfo-SMCC, dissolved in 0.1 ml dimethyl sulfoxide, were added to the protein solution. Reaction of carrier protein activation was carried out for 2 hours at room temperature. Excessive sulfo-SMCC was removed by gel-filtration using NAP-5 columns (obtained from GE Healthcare Life Sciences, Piscataway, NJ). NAP-5 columns were pre-equilibrated with 10 mM KH2PO4, 150 mM NaCl, pH 7.2. Then 2 mg of synthetic peptide-1 or peptide-2 were added to protein solution to start the conjugation. This reaction was carried out for 2 hours on ice with constant shaking. Unreacted peptide fraction was removed from protein-peptide conjugate by using gel-filtration NAP-5 columns, pre-equilibrated with PBS. The conjugation of the peptides to appropriate carrier protein was confirmed by 3-5 kDa increase in the protein molecular weight revealed by using sodium dodecyl sulphate poly-acrylamide gel electrophoresis. Conjugates were aliquoted and stored at -20°C before use.

The BSA-synthetic peptide was diluted to a 5 $\mu$g/mL solution with 0.1 M NaHCO$_3$ (pH 9.6), added to a highly adsorbed 96-well plate at 100 $\mu$L per well, and incubated overnight at 4°C. The solution was dried, and added 400 $\mu$L of PBS solution containing 1% BSA to each well, and standed for 3 hours at room temperature for blocking. The solution was dried and washed three times with PBS containing 0.05% Tween-20. The 11 antibodies were diluted to 100 $\mu$g/mL in PBS containing 1% BSA, 100 $\mu$L was added to each well, and incubated at 37°C for 1.5 hours. The solution was dried and washed 3 times with PBS containing 0.05% Tween-20. Prepare a 1/2000-fold dilution of goat anti-mouse IgG-HRP solution in PBS solution containing 1% BSA, added 100 $\mu$L of the solution to each well, which was incubated at 37°C for 1.5 hours. The solution was dried and washed 3 times with PBS containing 0.05% Tween-20. 100$\mu$L of TMB substrate solution was added to each well, incubated at room temperature for 30 minutes, and terminated with 100$\mu$L of 0.16M sulfuric acid solution. The absorbance at OD450 is obtained on a microplate reader.

According to the above method, 11 antibodies were detected, and the results were shown as follows:
NA2 recognized peptide with 78-93 amino acid residues (a.a.r) and peptide with 83-100 a.a.r, thus the epitope for NA2 is within 83- 93 a.a.r.
NA3 and NA5 recognized peptide with 39-54 a.a.r, thus the epitope for NA3 and NA5 is within 39-54 a.a.r.
NA4 and NA8 recognized peptide with 22-40 a.a.r, thus the epitope for NA4 and NA8 is within 22-40 a.a.r.
NA6 and NA9 recognized peptide with 161-178 a.a.r, thus the epitope for NA6 and NA9 is within 161-178 a.a.r.
NA7 and NA10 recognized peptide with 174-192 a.a.r, thus the epitope for NA7 and NA10 is within 174-192 a.a.r.
NA11 and NA12 recognized peptide with 174-192 a.a.r and peptide with 182-197 a.a.r, thus the epitope for NA11 and NA12 is within 182-192 a.a.r.

Example 5. Sandwich immunoassay using antibody pair

To find the best antibody pair, a sandwich immunoassay was used. The detection ability of human cardiac troponin was tested by combining antibodies that recognize different epitopes. In addition to the antibodies used in the present disclosure, KA1(known antibody 1), KA2(known antibody 2), and KA3(known antibody 3)(cat# RC4T21) antibodies from HyTest were used, and the sequence of those antibodies are shown in Table 3(amino acid sequence) below.

Table 3

| Antibody name | Heavy chain | | |
|---|---|---|---|
| | CDR1 | CDR2 | CDR3 |
| KA1 | GIDLGSFA | INSAGIL | ARLYDL |
| KA2 | GIDLAGYA | IASDGEK | ARFYDL |
| KA3 | GIDLGSFA | INSEDIL | ARFWDL |
| | Light chain | | |
| | CDR1 | CDR2 | CDR3 |
| KA1 | QSVYDNNA | DASKLAS | LGDYDCSSADCYA |
| KA2 | ESVYDNNA | DASKVAS | LGDYDCDSGDCYV |
| KA3 | QSVYDGTA | DASKLAS | LGDYDCSSGDCYV |

The detection ability of antibody pairs was determined using dissociation-enhanced lanthanide fluorescent immunoassays and chemiluminescence immunoassays.

To perform sandwich fluorescent immunoassays, detection MAbs labeled with stable Eu3+ chelate were used as described by Hyytiä et al. (2010). Capture antibodies in this assay were unlabeled. Capture antibodies 100 μL per well (2 μg/ml) in PBS, were incubated in 96-well immunoassay plates for 30 min at room temperature upon constant shaking. The plates were washed with 10 mM Tris-HCl (pH 7.8) buffer, which was supplemented by 0.15 M NaCl, 0.025 % Tween 20 and 0.5 g/L NaN3 (Washing Solution, WS). After washing with 0.05 ml of buffer B (50 mM Tris-HCl buffer, pH 7.8, 0.9 % NaCl, 0.01 % Tween 40, 0.5% BSA and 0.05 % NaN3), the solution (2mg/L) of detection antibodies were added to the plates. Immediately after detection 25μL antigen (native human cardiac troponin complex) standard solutions in 50 mM Tris-HCl buffer, pH 7.8, 150mM KCl, 5mM $CaCl_2$, 7.5% BSA and 0.15% $NaN_3$ were added. The plates were incubated for 30 min at room temperature with constant shaking. After washing, 0.1ml of DELFIA® Enhancement solution (Perkin Elmer, Finland) per well were added and incubated for 10 min at room temperature with gentle shaking. Fluorescence of $Eu^{3+}$ was measured on a Victor 1420 multilabel counter (Wallac-Perkin Elmer, Finland). The fluorescence was expressed in counts per second (CPS).

To perform sandwich chemiluminescent immunoassays (CLIA), detection MAbs were labeled with biotin derivative. Capture antibodies (unlabeled), 100 μL per well (2 μg/ml) in phosphate buffer saline (PBS), were incubated in 96-well immunoassay plates for 30 min at room temperature with constant shaking. The plates were washed with PBS supplemented by 0.025 % Tween 20 and 0.05 % ProClin (PBST). After washing with 0.05 ml of the buffer B, a solution (2 mg/L) of detection antibodies in CLIA assay buffer (PBST, containing 75 g/L BSA) was added to the plates. Immediately after detection, 25 μL antigen (native hc troponin complex) standard solutions in 50 mM Tris-HCl buffer, pH 7.8, 150mM KCl, 5mM $CaCl_2$, 7.5% BSA and 0.15% $NaN_3$ were added. The plates were incubated for 30 min at room temperature with constant shaking. After washing with PBST, 0.1 ml of Pierce™ Streptavidin Poly-HRP per well (5 ng/ml in CLIA assay buffer) were added and incubated for 5 min at room temperature with gentle shaking. After washing with PBST (6 times) the SuperSignal™ ELISA Femto Substrate was added. Incubation time was 1 min, at room temperature with gentle shaking. Chemiluminescence was measured on a EnVision 2105 multimode plate reader (Perkin Elmer, Finland). The chemiluminescence was expressed in counts per second (CPS).

Sandwich immunoassays were able to detect only hcTnI and had no cross-reaction (or less than 0.03%) with skeletal isoforms of TnI.

Limit of Blank (LOB) and LOD determination were performed according to Clinical and Laboratory Standard Institute (CLSI) recommendations. The measurement results are shown in Tables 4 and 5.

It shows that the sensitivity of these antibody pairs is below 10pg/ml, which can meet the performance requirements for the development of high-sensitivity troponin kits.

Table 4. LOD of Antibody Pairs in DELFIA

| Capture antibody | Dection antibody | LOD, pg/ml |
|---|---|---|
| KA2 | NA10 | 2.0 |
| KA2 | NA7 | 0.9 |
| NA4 | NA12 | 0.7 |
| NA5 | NA6 | 0.8 |

(continued)

| Capture antibody | Dection antibody | LOD, pg/ml |
|---|---|---|
| KA1 | NA6 | 2.5 |
| KA3 | NA9 | 4.3 |
| KA3 | NA12 | 2.4 |
| NA2 | NA10 | 3.3 |
| NA4 | NA6 | 2.4 |

Table 5. LOD of Antibody Pairs in CLIA

| Capture antibody | Dection antibody | LOD, pg/ml |
|---|---|---|
| KA2 | NA10 | 1.0 |
| NA3 | NA9 | 0.8 |
| NA11 | NA9 | 0.9 |
| NA12 | NA9 | 1.3 |
| NA2 | NA9 | 1.0 |

Example 6. Sex-specific 99th percentile of hs-cTnI specific immunoassay

To determine the clinical performance of hs-cTnI specific sandwich chemiluminescent immunoassays, the sex-specific 99th percentile was determined according to Clinical and Laboratory Standard Institute (CLSI) recommendations (Defining, Establishing, and Verifying Reference Intervals in the Clinical Laboratory; Approved Guideline-Third Edition; EPC28-A3 Vol.28 NO: 30).

Briefly, serum or plasma samples from both 400 healthy males and 400 healthy females (people with NT-proBNP$\geq$ 125ng/L, HbA1c>6.5%, eGFR<60mL/min were excluded) were tested by NA2/NA9 pair of "sandwich"-immunoassay on Mindray chemiluminescent analyzer. The detection results are shown in Table 6 below, and it can be seen from the table that high-sensitivity detection can be achieved in both males and females.

Table 6. sex specific 99th percentile for NA2/NA9 pair in CLIA

| th 99 percentile | cTnI Value (ng/L) | Rule-out rate (%) |
|---|---|---|
| Total | 26.5 | 75% |
| Female | 17.6 | 65% |
| Male | 33.7 | 80% |

Example 7. Clinical diagnosis of different myocardial injuries by hs-cTnI specific immunoassay

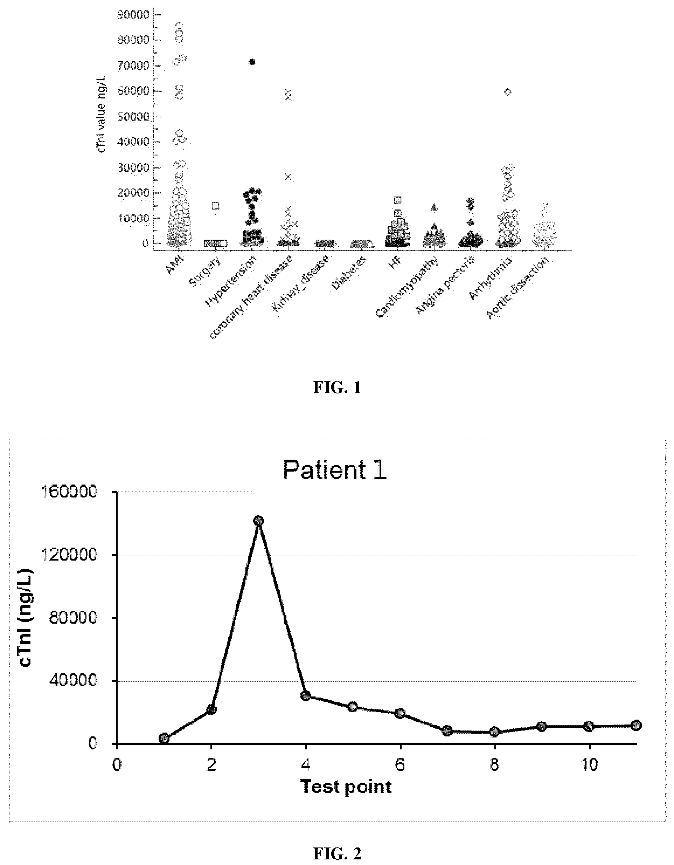

To determine the diagnosis performance of hs-cTnI sandwich immunoassays, serum or plasma samples from patients with different myocardial injury (including acute myocardial infarction (AMI), myocardial surgery, coronary artery disease, heart failure, cardiomyopathy, angina pectoris, arrhythmia, aortic dissection, etc.) and non-myocardial injury patients (including renal disease, diabetes, etc.) were evaluated. The results showed that cardiac troponin could not be detected in samples from the patient with kidney disease and diabetes, while cardiac troponin could be detected in the rest of the patient samples related to myocardial injury, as shown in FIG. 1. Therefore, the hs-cTnI assay could significant differentiate the myocardial injury diseases with other non-myocardial diseases.

Example 8. Prognosis performance of acute myocardial injury patients by hs-cTnI specific immunoassay

To determine the prognostic performance of the hs-cTnI sandwich immunoassay, serum or plasma samples from different acute myocardial infarction patients at different time points were dynamically monitored. The results showed that hs-cTnI values were highly consistent with disease stage progression. Details are provided as follows:

Patient 1: 11 yr female. When she was senting to ICU due to ventricular fibrillation, arrhythmia and respiratory arrest, the first test was carried out using antibody pair KA2/NA10 (test 1); the second test was carried out when she was receiving ontinuous cardiopulmonary resuscitation and ECG monitoring (test 2),. The result showed that cardiac troponin elevated indicating myocardial injury. After she arrived into ICU, defibrillation was operated immediately. Then sinus rhythm was restored, then she received tracheal intubation, entilator-assisted breathing, arteriovenous catheter and ECMO. The 3rd test was performed before treatment (test 3), while her myocardial injury was more severe. During treating process, subsequent tests 4-11(test 4-11) showed that the value of cardiac troponin continued to decline, as shown in FIG. 2. These tests were carried out by KA2/NA10 pair assay.

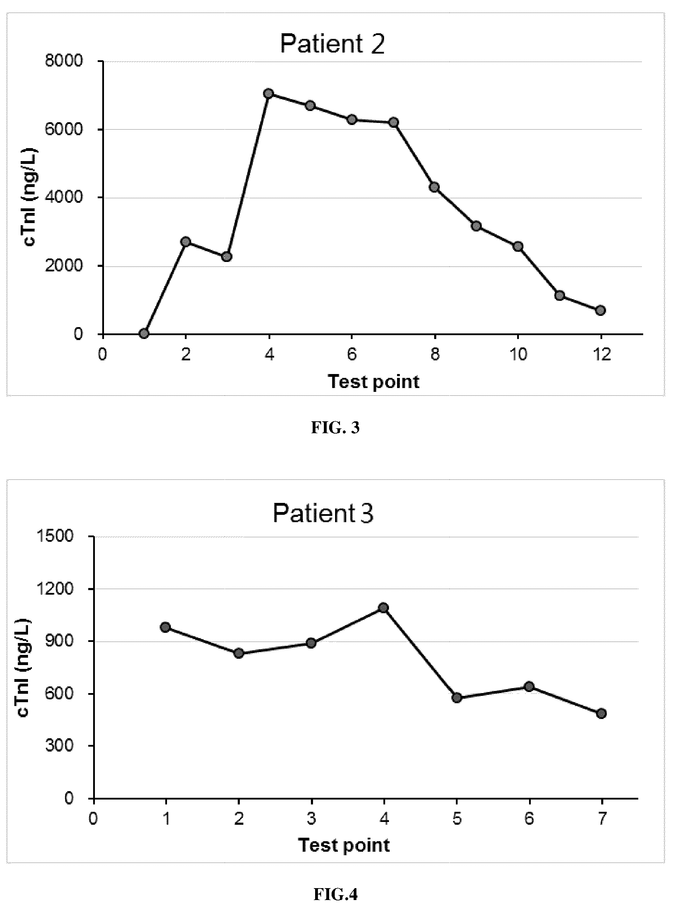

Patient 2: 57 yr female with a history of hypertension and jaundice hepatitis. She was sent to ED and diagnosed as aortic dissection, the cTn was positive using antibody pair NA4/NA6 (test 1). She had a series of surgeries that induced myocardial injury, released amount of cTnI was significantly elevated(test2). An operation for the prevention of venous thromboembolism was carried out, the cTnI was stable (test3). Chest surgery was carried out again, cTnI was significantly elevated again (test 4-5). Massive embolism of left cerebral hemisphere during the sixth test, but the cTnI was stable. Then a brain surgery was performed, the blood circulation was stable, and cTnI slightly decreased (test 7-8). After the brain surgery, the heart and kidney functions were stable, and cTnI was decreasing (test 9-12), as shown in FIG. 3. These tests were carried out by NA4/NA6 pair assay.

Patient 3: A patient with a history of hypertension and oral cancer. He was sent to ED and diagnosed as NSTEAMI. At this time, the first test is carried out using antibody pair NA11/NA9 (test 1), the cTnI was very high. After being received a series of treatments and drugs to control hypertension, heart rate and coagulation, the second test is carried out for the patient (test 2), the cTnI in the second test is lower than the first test. After cardio surgery that induced myocardial damage, cTnI increased in the third and fourth tests (test 3-4). A series of treatments and drugs were administered to control hypertension, heart rate and coagulation. The ischemia was slowly recovered, and then the cTnI results were decreasing from the fifth test to the seventh test (test 5-7), as shown in FIG. 4. These tests were carried out by NA11/NA9 pair assay.

Example 9. Measurement of the specificity of antibodies

In order to evaluate the potential cross-reactivity of antibodies, the following substances were added at a concentration ( 1000ng/ml) indicated to a sample with known cTnI concentration (0.7ng/L). Results from the spiked samples were compared with those of unspiked control samples. All antibodies show high specificity for cTnI. Percent cross-reactivity is calculated as:

$$\% \text{ cross-reactivity} = \frac{(\text{concentration of spiked sample} - \text{concentration of unspiked sample})}{\text{concentration of substance}} \times 100.$$

Table 6

| Substance | Concentration | NA2 | NA3 | NA4 | NA5 | NA6 | NA7 | NA8 | NA9 | NA10 | NA11 | NA12 |
|---|---|---|---|---|---|---|---|---|---|---|---|---|
| | (ng/mL) | \% cross-reactivity | | | | | | | | | | |
| Skeletal troponin I | 1000 | ND[a] | ND[a] | ND[a] | NDa | ND[a] | ND[a] | ND[a] | ND[a] | ND[a] | ND[a] | ND[a] |
| Skeletal troponin T | 1000 | ND[a] | ND[a] | ND[a] | ND[a] | ND[a] | ND[a] | ND[a] | ND[a] | ND[a] | ND[a] | ND[a] |
| Cardiac troponin T | 1000 | 6.64 E-04 | 8.00 E-04 | 3.10 E-04 | 7.18 E-04 | 2.30 E-04 | 1.20 E-04 | 6.71 E-04 | 4.00 E-04 | 2.20 E-04 | 3.06 E-04 | 8.01 E-04 |
| Troponin C | 1000 | 8.00 E-04 | 5.00 E-04 | 2.80 E-04 | 2.00 E-04 | 2.20 E-05 | 7.00 E-04 | 1.40 E-05 | 9.00 E-04 | 1.40 E-04 | 2.36 E-04 | 4.40 E-04 |
| Actin | 1000 | NDa | NDa | NDa | NDa | NDa | NDa | NDa | NDa | NDa | NDa | NDa |
| Tropomyosin | 1000 | 2.58 E-04 | 1.20 E-04 | 1.89 E-04 | 2.72 E-04 | 1.90 E-04 | 1.57 E-04 | 2.55 E-05 | 1.40 E-04 | 1.25 E-04 | 2.06 E-04 | 4.02 E-04 |
| Myosin light chain | 1000 | ND[a] | ND[a] | ND[a] | ND[a] | ND[a] | ND[a] | ND[a] | ND[a] | ND[a] | ND[a] | ND[a] |

(continued)

| Substance | Concentration | NA2 | NA3 | NA4 | NA5 | NA6 | NA7 | NA8 | NA9 | NA1 0 | NA1 1 | NA1 2 |
|---|---|---|---|---|---|---|---|---|---|---|---|---|
| | (ng/mL) | % cross-reactivity | | | | | | | | | | |
| Myoglobin | 1000 | ND$^a$ | ND$^a$ | ND$^a$ | ND$^a$ | ND$^a$ | ND$^a$ | ND$^a$ | ND$^a$ | ND$^a$ | ND$^a$ | ND$^a$ |
| CK-MB | 1000 | ND$^a$ | ND$^a$ | ND$^a$ | ND$^a$ | ND$^a$ | ND$^a$ | ND$^a$ | ND$^a$ | ND$^a$ | ND$^a$ | ND$^a$ |

[00120] ND$^a$= Not Detectable.

**Claims**

1. An isolated antibody or antigen-binding fragment thereof that specifically binds to cardiac troponin I, comprising a heavy chain and a light chain, wherein the heavy chain comprises a heavy chain variable region, and the light chain comprises a light chain variable region; wherein, the heavy chain variable region comprises:

   a. CDR-H1, having an amino acid sequence selected from a group of SEQ ID NOs: 1~11;
   b. CDR-H2, having an amino acid sequence selected from a group of SEQ ID NOs: 11~22; and
   c. CDR-H3, having an amino acid sequence selected from a group of SEQ ID NOs: 23~33; and

   the light chain variable region comprises:

   a. CDR-L1, having an amino acid sequence selected from a group of SEQ ID NOs: 34~44;
   b. CDR-L2, having an amino acid sequence selected from a group of SEQ ID NOs: 45~55; and
   c. CDR-L3, having an amino acid sequence selected from a group of SEQ ID NOs: 56~66.

2. The isolated antibody or antigen-binding fragment thereof according to claim 1, comprising:

   CDR-H1 as shown in SEQ ID NO: 1, CDR-H2 as shown in SEQ ID NO: 12, and CDR-H3 as shown in SEQ ID NO: 23; and CDR-L1 as shown in SEQ ID NO: 34, CDR-L2 as shown in SEQ ID NO: 45, and CDR-L3 as shown in SEQ ID NO: 56; or
   CDR-H1 as shown in SEQ ID NO: 2, CDR-H2 as shown in SEQ ID NO: 13, and CDR-H3 as shown in SEQ ID NO: 24; and CDR-L1 as shown in SEQ ID NO: 35, CDR-L2 as shown in SEQ ID NO: 46, and CDR-L3 as shown in SEQ ID NO: 57; or
   CDR-H1 as shown in SEQ ID NO: 3, CDR-H2 as shown in SEQ ID NO: 14, and CDR-H3 as shown in SEQ ID NO: 25; and CDR-L1 as shown in SEQ ID NO: 36, CDR-L2 as shown in SEQ ID NO: 47, and CDR-L3 as shown in SEQ ID NO: 58; or
   CDR-H1 as shown in SEQ ID NO: 4, CDR-H2 as shown in SEQ ID NO: 15, and CDR-H3 as shown in SEQ ID NO: 26; and CDR-L1 as shown in SEQ ID NO: 37, CDR-L2 as shown in SEQ ID NO: 48, and CDR-L3 as shown in SEQ ID NO: 59; or
   CDR-H1 as shown in SEQ ID NO: 5, CDR-H2 as shown in SEQ ID NO: 16, and CDR-H3 as shown in SEQ ID NO: 27; and CDR-L1 as shown in SEQ ID NO: 38, CDR-L2 as shown in SEQ ID NO: 49, and CDR-L3 as shown in SEQ ID NO: 60; or
   CDR-H1 as shown in SEQ ID NO: 6, CDR-H2 as shown in SEQ ID NO: 17, and CDR-H3 as shown in SEQ ID NO: 28; and CDR-L1 as shown in SEQ ID NO: 39, CDR-L2 as shown in SEQ ID NO: 50, and CDR-L3 as shown in SEQ ID NO: 61; or
   CDR-H1 as shown in SEQ ID NO: 7, CDR-H2 as shown in SEQ ID NO: 18, and CDR-H3 as shown in SEQ ID NO: 29; and CDR-L1 as shown in SEQ ID NO: 40, CDR-L2 as shown in SEQ ID NO: 51, and CDR-L3 as shown in SEQ ID NO: 62; or
   CDR-H1 as shown in SEQ ID NO: 8, CDR-H2 as shown in SEQ ID NO: 19, and CDR-H3 as shown in SEQ ID NO: 30; and CDR-L1 as shown in SEQ ID NO: 41, CDR-L2 as shown in SEQ ID NO: 52, and CDR-L3 as shown in SEQ ID NO: 63; or
   CDR-H1 as shown in SEQ ID NO: 9, CDR-H2 as shown in SEQ ID NO: 20, and CDR-H3 as shown in SEQ ID NO: 31; and CDR-L1 as shown in SEQ ID NO: 42, CDR-L2 as shown in SEQ ID NO: 53, and CDR-L3 as shown in SEQ ID NO: 64; or
   CDR-H1 as shown in SEQ ID NO: 10, CDR-H2 as shown in SEQ ID NO: 21, and CDR-H3 as shown in SEQ ID NO:

32; and CDR-L1 as shown in SEQ ID NO: 43, CDR-L2 as shown in SEQ ID NO: 54, and CDR-L3 as shown in SEQ ID NO: 65; or

CDR-H1 as shown in SEQ ID NO: 11, CDR-H2 as shown in SEQ ID NO: 22, and CDR-H3 as shown in SEQ ID NO: 33; and CDR-L1 as shown in SEQ ID NO: 44, CDR-L2 as shown in SEQ ID NO: 55, and CDR-L3 as shown in SEQ ID NO: 66.

3. The isolated antibody or antigen-binding fragment thereof according to claim 1, wherein, the antibody or antigen-binding fragment thereof has a KD value of less than 20 nM.

4. The isolated antibody or antigen-binding fragment thereof according to claim 3, wherein, the antibody or antigen-binding fragment thereof has a KD value of less than 5 nM;
preferably, the antibody or antigen-binding fragment thereof has a KD value of less than 2 nM.

5. An isolated nucleic acid molecule, comprising a nucleotide sequence encoding the antibody or antigen-binding fragment thereof according to any one of claims 1 to 4.

6. A vector, comprising the nucleic acid molecule according to claim 5.

7. A host cell, comprising the nucleic acid molecule according to claim 5 or the vector according to claim 6.

8. A conjugate, comprising a monoclonal antibody or antigen-binding fragment thereof and a coupling moiety, wherein the monoclonal antibody is the antibody or antigen-binding fragment thereof according to any one of claims 1 to 4, and the coupling moiety is a detectable label.

9. An antibody pair, comprising a capture antibody and a detection antibody, wherein the capture antibody and the detection antibody target different epitopes of cardiac troponin I, wherein the capture antibody and the detection antibody are selected from the group consisting of the antibody or antigen-binding fragment thereof according to any one of claims 1 to 4, Known Anitbody 1, Known Anitbody 2, or Known Anitbody 3 antibody, wherein,

the Known Anitbody 1 is an antibody which comprises CDR-H1~3 as shown in SEQ ID NOs: 67-69; and CDR-L1~3 as shown in SEQ ID NOs: 70-72;
the Known Anitbody 2 is an antibody which comprises CDR-H1~3 as shown in SEQ ID NOs: 73-75; and CDR-L1~3 as shown in SEQ ID NOs: 76-78;
the Known Anitbody 3 is an antibody which comprises CDR-H1~3 as shown in SEQ ID NOs: 79-81; and CDR-L1~3 as shown in SEQ ID NOs: 82~84.

10. The antibody pair according to claim 9, wherein, the antibody pair comprises a capture antibody and a detection antibody, wherein,

the capture antibody is an antibody which comprises CDR-H1~3 as shown in SEQ ID NOs: 73-75; and CDR-L1~3 as shown in SEQ ID NOs: 76-78; the detection antibody is an antibody which comprises CDR-H1 having an amino acid sequence of SEQ ID NO: 9 and CDR-H2 having an amino acid sequence of SEQ ID NO: 20, and CDR-H3 having an amino acid sequence of SEQ ID NO: 31; and CDR-L1 having an amino acid sequence of SEQ ID NO: 42, CDR-L2 having an amino acid sequence of SEQ ID NO: 53, and CDR-L3 having an amino acid sequence of SEQ ID NO: 64; or
the capture antibody is an antibody which comprises CDR-H1~3 as shown in SEQ ID NOs: 73-75; and CDR-L1~3 as shown in SEQ ID NOs: 76-78; the detection antibody is an antibody which comprises CDR-H1 having an amino acid sequence of SEQ ID NO: 6 and CDR-H2 having an amino acid sequence of SEQ ID NO: 17, and CDR-H3 having an amino acid sequence of SEQ ID NO: 28; and CDR-L1 having an amino acid sequence of SEQ ID NO: 39, CDR-L2 having an amino acid sequence of SEQ ID NO: 50, and CDR-L3 having an amino acid sequence of SEQ ID NO: 61; or
the capture antibody is an antibody which comprises CDR-H1~3 as shown in SEQ ID NOs: 67-69; and CDR-L1~3 as shown in SEQ ID NOs: 70-72; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60; or
the capture antibody is an antibody which comprises CDR-H1~3 as shown in SEQ ID NOs: 79-81; and CDR-L1~3 as shown in SEQ ID NOs: 82-84; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63; or

the capture antibody is an antibody which comprises CDR-H1~3 as shown in SEQ ID NOs: 79-81; and CDR-L1~3 as shown in SEQ ID NOs: 82-84; the detection antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66; or

the capture antibody is an antibody which comprises CDR-H1 having an amino acid sequence of SEQ ID NO: 3 and CDR-H2 having an amino acid sequence of SEQ ID NO: 14, and CDR-H3 having an amino acid sequence of SEQ ID NO: 25; and CDR-L1 having an amino acid sequence of SEQ ID NO: 36, CDR-L2 having an amino acid sequence of SEQ ID NO: 47, and CDR-L3 having an amino acid sequence of SEQ ID NO: 58; the detection antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66; or

the capture antibody is an antibody which comprises CDR-H1 having an amino acid sequence of SEQ ID NO: 4 and CDR-H2 having an amino acid sequence of SEQ ID NO: 15, and CDR-H3 having an amino acid sequence of SEQ ID NO: 26; and CDR-L1 having an amino acid sequence of SEQ ID NO: 37, CDR-L2 having an amino acid sequence of SEQ ID NO: 48, and CDR-L3 having an amino acid sequence of SEQ ID NO: 59; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60; or

the capture antibody is an antibody which comprises CDR-H1 having an amino acid sequence of SEQ ID NO: 1 and CDR-H2 having an amino acid sequence of SEQ ID NO: 12, and CDR-H3 having an amino acid sequence of SEQ ID NO: 23; and CDR-L1 having an amino acid sequence of SEQ ID NO: 34, CDR-L2 having an amino acid sequence of SEQ ID NO: 45, and CDR-L3 having an amino acid sequence of SEQ ID NO: 56; the detection antibody is an antibody which comprises CDR-H1 having an amino acid sequence of SEQ ID NO: 9 and CDR-H2 having an amino acid sequence of SEQ ID NO: 20, and CDR-H3 having an amino acid sequence of SEQ ID NO: 31; and CDR-L1 having an amino acid sequence of SEQ ID NO: 42, CDR-L2 having an amino acid sequence of SEQ ID NO: 53, and CDR-L3 having an amino acid sequence of SEQ ID NO: 64; or

the capture antibody is an antibody which comprises CDR-H1 having an amino acid sequence of SEQ ID NO: 3 and CDR-H2 having an amino acid sequence of SEQ ID NO: 14, and CDR-H3 having an amino acid sequence of SEQ ID NO: 25; and CDR-L1 having an amino acid sequence of SEQ ID NO: 36, CDR-L2 having an amino acid sequence of SEQ ID NO: 47, and CDR-L3 having an amino acid sequence of SEQ ID NO: 58; the detection antibody is an antibody which comprises CDR-H1 having an amino acid sequence of SEQ ID NO: 5 and CDR-H2 having an amino acid sequence of SEQ ID NO: 16, and CDR-H3 having an amino acid sequence of SEQ ID NO: 27; and CDR-L1 having an amino acid sequence of SEQ ID NO: 38, CDR-L2 having an amino acid sequence of SEQ ID NO: 49, and CDR-L3 having an amino acid sequence of SEQ ID NO: 60; or

the capture antibody is an antibody which comprises CDR-H1 having an amino acid sequence of SEQ ID NO: 2 and CDR-H2 having an amino acid sequence of SEQ ID NO: 13, and CDR-H3 having an amino acid sequence of SEQ ID NO: 24; and CDR-L1 having an amino acid sequence of SEQ ID NO: 35, CDR-L2 having an amino acid sequence of SEQ ID NO: 46, and CDR-L3 having an amino acid sequence of SEQ ID NO: 57; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63; or

the capture antibody is an antibody which comprises CDR-H1 having an amino acid sequence of SEQ ID NO: 10 and CDR-H2 having an amino acid sequence of SEQ ID NO: 21, and CDR-H3 having an amino acid sequence of SEQ ID NO: 32; and CDR-L1 having an amino acid sequence of SEQ ID NO: 43, CDR-L2 having an amino acid sequence of SEQ ID NO: 54, and CDR-L3 having an amino acid sequence of SEQ ID NO: 65; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63; or
the capture antibody is an antibody which comprises CDR-H1 having an amino acid sequence of SEQ ID NO: 11 and CDR-H2 having an amino acid sequence of SEQ ID NO: 22, and CDR-H3 having an amino acid sequence of SEQ ID NO: 33; and CDR-L1 having an amino acid sequence of SEQ ID NO: 44, CDR-L2 having an amino acid sequence of SEQ ID NO: 55, and CDR-L3 having an amino acid sequence of SEQ ID NO: 66; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63; or
the capture antibody is an antibody which comprises CDR-H1 having an amino acid sequence of SEQ ID NO: 1 and CDR-H2 having an amino acid sequence of SEQ ID NO: 12, and CDR-H3 having an amino acid sequence of SEQ ID NO: 23; and CDR-L1 having an amino acid sequence of SEQ ID NO: 34, CDR-L2 having an amino acid sequence of SEQ ID NO: 45, and CDR-L3 having an amino acid sequence of SEQ ID NO: 56; the detection antibody is an antibody which comprises CDR-H1 having an amino acid sequence of SEQ ID NO: 8 and CDR-H2 having an amino acid sequence of SEQ ID NO: 19, and CDR-H3 having an amino acid sequence of SEQ ID NO: 30; and CDR-L1 having an amino acid sequence of SEQ ID NO: 41, CDR-L2 having an amino acid sequence of SEQ ID NO: 52, and CDR-L3 having an amino acid sequence of SEQ ID NO: 63.

11. A kit, comprising the antibody or antigen-binding fragment thereof according to any one of claims 1 to 4, or the conjugate according to claim 8; preferably, wherein the kit further comprises a second antibody, which specifically recognizes the antibody or antigen-binding fragment thereof according to any one of claims 1 to 4.

12. A kit, comprising the antibody pair according to claim 9 or 10.

13. The antibody or antigen-binding fragment thereof according to any one of claims 1 to 4 or the antibody pair according to claim 9 or 10 or the conjugate according to claim 8 for use in detecting the presence or level of cardiac troponin I in a sample from a subject.

14. The antibody or antigen-binding fragment thereof according to any one of claims 1 to 4 or the antibody pair according to claim 9 or 10 or the conjugate according to claim 8 for use in assisting diagnosis of myocardial injury.

15. An antibody or antigen-binding fragment thereof that specifically binds to cardiac troponin I, wherein the antibody or antigen-binding fragment thereof is:

produced based on *Escherichia coli* Rosetta™(DE3)pLysS the plasmid of new antibody2 (NA2) deposited with the All-Russian National Collection of Industrial Microorganisms (VKPM) having Accession Number B-14631 on December 14, 2023;
produced based on *Escherichia coli* Rosetta™(DE3)pLysS 3 (NA3) VL deposited with VKPM having Accession Number B-14669 on February 7, 2024, and *Escherichia coli* RosettaTM(DE3)pLysS 3 (NA3) VH deposited with VKPM having Accession Number B-14668 on February 7, 2024;
produced based on *Escherichia coli* Rosetta™(DE3)pLysS 4 (NA4) VL deposited with VKPM having Accession Number B-14671 on February 7, 2024, and *Escherichia coli* RosettaTM(DE3)pLysS 4 (NA4) VH deposited with VKPM having Accession Number B-14670 on February 7, 2024;
produced based on *Escherichia coli* Rosetta™(DE3)pLysS 5 (NA5) VL deposited with VKPM having Accession Number B-14673 on February 7, 2024, and *Escherichia coli* RosettaTM(DE3)pLysS 5 (NA5) VH deposited with VKPM having Accession Number B-14672 on February 7, 2024;
produced based on *Escherichia coli* Rosetta™(DE3)pLysS 6 (NA6) VL deposited with VKPM having Accession Number B-14675 on February 7, 2024, and *Escherichia coli* RosettaTM(DE3)pLysS 6 (NA6) VH deposited with VKPM having Accession Number B-14674 on February 7, 2024;
produced based on *Escherichia coli* Rosetta™(DE3)pLysS 7 (NA7) VL deposited with VKPM having Accession Number B-14677 on February 7, 2024, and *Escherichia coli* RosettaTM(DE3)pLysS 7 (NA7) VH deposited with VKPM having Accession Number B-14676 on February 7, 2024;
produced based on *Escherichia coli* Rosetta™(DE3)pLysS 8 (NA8) VL deposited with VKPM having Accession Number B-14679 on February 7, 2024, and *Escherichia coli* RosettaTM(DE3)pLysS 8 (NA8) VH deposited with VKPM having Accession Number B-14678 February 7, 2024;
produced based on *Escherichia coli* Rosetta™(DE3)pLysS the plasmid of new antibody 9 (NA9) deposited with the VKPM having Accession Number B-14632 on December 14, 2023;
produced based on *Escherichia coli* Rosetta™(DE3)pLysS the plasmid of new antibody 10 (NA10) deposited with the VKPM having Accession Number B-14633 on December 14, 2023;

produced based on *Escherichia coli* Rosetta™(DE3)pLysS 11 (NA11) VL deposited with VKPM having Accession Number B-14681 on February 7, 2024, and *Escherichia coli* RosettaTM(DE3)pLysS 11 (NA11) VH deposited with VKPM having Accession Number B-14680 on February 7, 2024;

produced based on *Escherichia coli* Rosetta™(DE3)pLysS 12 (NA12) VL deposited with VKPM having Accession Number B-14683 on February 7, 2024, and *Escherichia coli* RosettaTM(DE3)pLysS 12 (NA12) VH deposited with VKPM having Accession Number B-14682 on February 7, 2024.

**FIG. 1**

**FIG. 2**

FIG. 3

FIG.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410389129X

- US 7285418 B

**Non-patent literature cited in the description**

- **CUMMINS et al.** *Am Heart Journal*, 1987, vol. 113, 1333-1344
- **BODAR et al.** *Clinical Chemistry*, 1992, vol. 38, 2203-2214

- **WARD et al.** *Nature*, 1989, vol. 341, 544-546
- **CACECI et al.** *Byte*, 1984, vol. 9, 340-362